(19) Europäisches Patentamt
European Patent Office
Office européen des brevets

(11) Publication number: **0 242 855**
**A2**

(12) **EUROPEAN PATENT APPLICATION**

(21) Application number: 87105854.1

(22) Date of filing: 21.04.87

(51) Int. Cl.³: A 61 K 7/48
A 61 K 9/00, A 23 L 1/03

(30) Priority: 21.04.86 JP 91769/86

(43) Date of publication of application:
28.10.87 Bulletin 87/44

(84) Designated Contracting States:
AT BE CH DE ES FR GB IT LI NL SE

(71) Applicant: LION CORPORATION
3-7, Honjo 1-chome
Sumida-ku Tokyo(JP)

(72) Inventor: Nagaoka, Yoshio
No. 1-17-9-102, Higashiyotsugi Katsushika-ku
Tokyo(JP)

(72) Inventor: Yasumasu, Tomoko
No. 500-1-3-309, Hongo-cho
Funabashi-shi Chiba-ken(JP)

(72) Inventor: Noda, Seiji
No. 531-464, Wakamatsu-cho
Chiba-shi Chiba-ken(JP)

(72) Inventor: Higo, Moriaki
No. 6-8-8, Makuharinishi
Chiba-shi Chiba-ken(JP)

(74) Representative: Kuhnen, Wacker & Partner
Schneggstrasse 3-5 Postfach 1729
D-8050 Freising(DE)

(54) **Crosslinking reaction inhibitor for collagen.**

(57) The present invention relates to a crosslinking inhibitor which inhibits the formation of crosslinking (aged crosslinking) of collagen by utilizing the Maillard reaction between collagen and sugar. The inhibitor comprises a silicon compound having at least one silanol group in its molecule, such as methyl silanetriol, dimethyl silanediol, trimethyl silanol, dodecyl silanetriol, cycrohexyl silanetriol or propenyl silanetriol.

FIG. 1

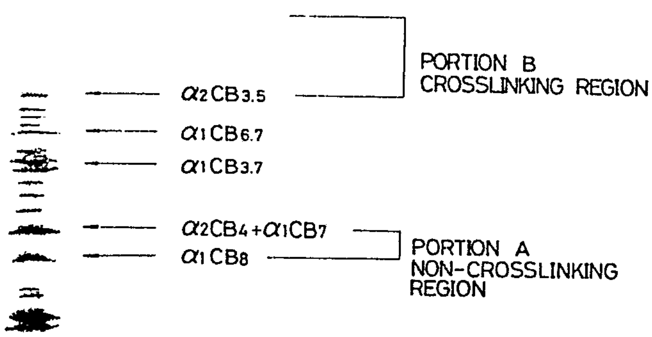


EP 0 242 855 A2

SPECIFICATION

## Title of the Invention

Crosslinking Reaction Inhibitor for Collagen

## Background of the Invention

### (1) Field of the Invention

The present invention relates to a crosslinking inhibitor which inhibits the formation of crosslinking (aged crosslinking) of collagen by utilizing the Maillard reaction between collagen and sugar.

### (2) Prior Art

It has been previously known that amino acids added to foods and medicines, such as lysine, glutamic acid and alanine, react with sugars such as galactose and dextrose to produce a brown substance, this reaction being called the Maillard reaction. Techniques for inhibiting the above-described browning and inhibiting the production of discoloration over long-term preservation period have therefor been developed. For example, Japanese Patent Un-examined Publication No. 129945/1983 discloses a technique in which a thin film cover of a particular adhesive substance is provided on the periphery of at least one of granular sugar and amino acid and Japanese Patent Un-examined Publication No. 164683/1983 discloses a composition to which a polysaccharide containing 3% or less of water is added in an amount of 40% or more with respect to an amino acid.

On the other hand, evidence has been found that the hemoglobin of a persons afflicted with diabates mellitus and the collagen of aged persons are subjected to the Maillard reaction and the correlation between the Maillard reaction and diabetes or senescence has attracted considerable attention (with regard to diabetes, S. Rahbar; Chin. Chim. Acta, 22, 296 (1968) and with regard to senescence, D. Fujimoto; Biomedical Res., 5, 279 (1984)). Since these studies relate to proteins in organisms and the above-described method cannot inhibit the Maillard reaction that is produced in organisms, it has been hoped that a novel technique might be devleoped.

Meanwhile, GB2159408A (U.S.SN 712819) discloses a humectant which comprises (A) a silcion compound having at least one silanol group in the molecular thereof and (B) a water-soluble polymer substance which is most suitably a mucopolysaccharide, protein, cellulose derivative, starch derivative or polyvinyl alcohol.

## Summary of the Invention

The inventors focused their attention on collagen of the skin and investigated the inhibition effect of collagen on the Maillard reaction (aged crosslinking) using various compounds. As a result, he achieved the present invention on the basis of the finding that a silicon compound having silanol groups in its molecule exhibits the above-described effect.

It is therefore a primary object of the present

invention to provide a novel technique which is capable of effectively inhibiting the Maillard reaction produced in organisms, particularly the crosslinking reaction of collagen. Another object of the present invention is to provide a technique which is capable of inhibiting the crosslinking reaction of collagen so as to inhibit the senescence of connective tissue.

These and other objects of the present invention will be clear from the following description.

In accordance with the present invention, there is provided a crosslinking reaction inhibitor for collagen, which comprises a silicon compound having at least one silanol group in its molecule.

## Brief Description of the Drawings

Fig. 1 shows the gel electrophoresis of the CB peptides of collagen; and

Fig. 2 shows the densitometry of the gel electrophoresis.

## Description of the Preferred Embodiments

The silicon compound used in the present invention may be any compound having at least one silanol group in its molecule, and it is preferable to use a compound having the formula (I):

$$R_nSi(OH)_{4-n} \qquad (I)$$

wherein R is an alkyl, aralkyl, alkenyl, cycloalkyl, or alkoxy

gorup of 1 to 12 carbon atoms, preferably 1 to 6 carbon atoms, two or more of R are the same or different from each other, and n is an integer of between 0 and 3, preferably between 1 and 3 or a dehydrating condensation product thereof. Examples of such silicon compounds include one or mixtures of two or more of methyl silanetriol, dimethyl silanediol, trimethyl silanol, ethyl silanetriol, diethyl silanediol, propyl silanetriol, octyl silanetriol, dodecyl silanetriol, benzyl silanetriol, phenylethyl silanetriol, propenyl silanetriol, cyclohexyl silanetriol, methoxy silanetriol, and ethoxy silanetriol. It is also possible to use a dimer or trimer produced by the dehydrating condensation of methyl silanetriol. Among the above-described compounds, it is particularly preferable in the present invention to use methyl silanetriol.

The above-described silicon compounds having silanol groups in their molecules can be produced by the following method:

(i) The corresponding chlorosilanes are hydrolyzed and reacted with alkali hydroxide to obtain siliconates which show pH values adjusted to between 4 and 5 by a cation exchange resin or various acids, or

(ii) the corresponding alkoxysilanes are hydrolyzed.

These silicon compounds can be used as an inhibitor

for the crosslinking formation of collagen as they are or in the state of being dissolved or dispersed in a solvent such as water or ethanol. In addition, it is possible to add various additives such as an antiseptic or a perfume to the inhibitor for the formation of crosslinking.

The silicon compound used in the present invention involves no problem with respect to safety. Shown below are data as to the safety of the silicon compounds.

| | LD50 (mg/kg) |
|---|---|
| Methyl silanetriol (Rat subcutaneous treatment) | 1,000 or more |

The present invention is capable of effectively preventing the corsslinking of collagen by sugars. Therefore, the use of the inhibitor of the present invention is able to effectively prevent various types of senescence such as skin senescence produced by the crosslinking of collagen present in the human body.

Thus, the crosslinking inhibitor of the present invention can be used as a skin preparation such as a cosmetic or ointment, or in a medical treatment such as a method involving injection, or by adding it to a health drink or a health food.

When the inhibitor is used in a skin preparation, it is preferable that the preparation contain 0.01 to 10 wt% (referred to as % hereinafter) of the inhibitor of the present

invention. When the inhibitor is used for medical treatment by injection or as a food, it is preferable to use the inhibitor in an amount of 0.1 to 10 mg and 0.01 to 1 mg relative to 1 kg of body weight, respectively.

The present invention is described in detail below with reference to unlimitative examples.

Example 1

Methyl silanetriol was produced by the following method and the inhibitor of the present invention containing the methyl silanetriol produced was added to collagen or a system comprising collagen and glucose in various amounts to form various samples, and the effects thereof in inhibiting the corsslinking of collagen were examined.

Method of producing methylsilanetriol

The precipitates produced by hydrolysis of methyltrichlorosilane were recovered and dissolved in a solution of sodium hydroxide in an amount which was 3 times that of the precipitates to prepare an aqueous solution of trisodium methylsiliconate.

This aqueous solution was diluted with water and the pH value thereof was adjusted to between 4 and 5 by a cation exchange resin or various acids to form an aqueous solution of methyl silanetriol.

Method of measuring the effect of crosslinking inhibition

100 mg of collagen obtained by grinding and deliming the bone of a spring chicken, 1 ml of 0.2M phosphate buffer solution (pH 7.4) of 0.2M glucose, and a given amount of the silicon compound were reacted at 37°C for 4 weeks. After the reaction was completed, the collagen was separated by filtering the reaction solution and 10 mg of the collagen was cut with bromine cyanide (CNBr) in accordance with a conventional method (the formation of CB peptides) and then subjected to acrylamide gel electrophoresis.

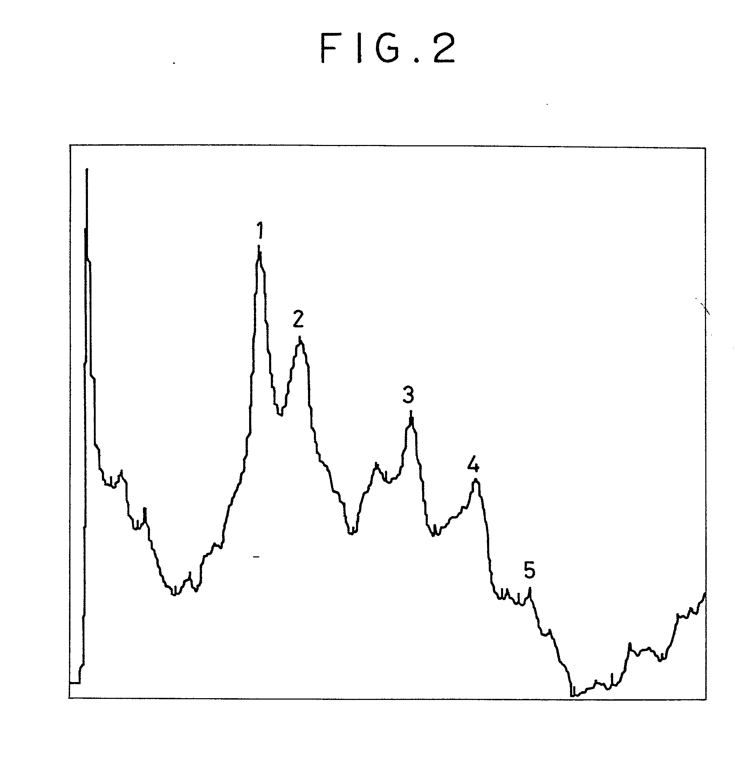

An example of the pattern obtained from the gel electrophresis of the CB peptides is shown in Fig. 1 and the result of the measurement by densitometry is shown in Fig. 2. Table 1 shows the ratios of portion A (a low-molecular weight region) to portion B (a high-molecular weight region: high-molecular substances of collagen having crosslinking formed by the Maillard reaction) in Fig. 1 and the rates of the collagen solubilized by the cutting with CNBr, together with the compositions of the samples used.

In Fig. 1, the collagen migrates from the upper portion B toward the lower portion A of the figure, the upper portion B being the crosslinking region and the lower portion A being the non-crosslinking region. The respective bands are identified as the peptides shown on the right side of the figure. In Fig. 2, the right side is the high-molecular weight region and the left side is the low-molecular weight region. Reference numbers in the figure correspond to those

of the main peaks of the gel electrophoresis in Fig. 1, they correspond to the following:

1 $\alpha_1CB_8$

2 $\alpha_2CB_4 + \alpha_1CB_7$

3 $\alpha_1CB_3$. 7

4 $\alpha_1CB_6$. 7

5 $\alpha_1CB_3$. 5

Table-1

| Sample No. | Composition of sample | | | Result | |
|---|---|---|---|---|---|
| | Collagen (mg) | Glucose (M) | Methyl-silanetriol (%) | Portion A/ portion B | Soluble collagen (%) |
| 1 | 100 | - | - | 0.11 | 100 |
| 2 | 100 | 0.2 | - | 0.53 | 54 |
| 3 | 100 | 0.2 | 0.003 | 0.55 | 50 |
| 4 | 100 | 0.2 | 0.01 | 0.40 | 81 |
| 5 | 100 | 0.2 | 0.03 | 0.35 | 86 |
| 6 | 100 | 0.2 | 0.1 | 0.40 | 76 |
| 7 | 100 | 0.2 | 0.3 | 0.54 | 54 |

It can be seen from the above results that the addition of 0.01 to 0.1% of methyl silanetriol inhibits the crosslinking and polymerization of collagen and thus silanol compounds have the effect of inhibiting the crosslinking. Since the concentration of collagen and glucose in the living body is lower than that of the evaluation method of the present invention, it is thought that the corsslinking takes place over a very long time and the proper amount of a silanol to be compound administered is not limited to the amount of 0.01 to 0.1% for which the effect in the present invention was demonstrated.

Example 2

One silicon compound was selected from the group consisting of dimethy silanediol, trimethyl silanol, dodecyl silanetriol, cycrohexyl silanetriol, propenyl silanetriol and a dimer of methy silanetriol, and the effects thereof in inhibiting the crosslinking of collagen were measured by the same method as set forth in Example 1. The results obtained are shown in Table 2.

Table 2

| Sample No. | Composition of sample | | | Result | |
|---|---|---|---|---|---|
| | Collagen (mg) | Glucose (M) | Silicon compound (%) | Portion A/ Portion B | Soluble collagen (%) |
| 1<br>2 | 100<br>100 | -<br>0.2 | -<br>- | 0.11<br>0.53 | 100<br>54 |
| 8<br>9 | 100<br>100 | 0.2<br>0.2 | Dimethyl silanediol 0.01<br>0.1 | 0.43<br>0.35 | 76<br>80 |
| 10<br>11 | 100<br>100 | 0.2<br>0.2 | Trimethyl silanol 0.01<br>0.1 | 0.45<br>0.38 | 70<br>75 |
| 12<br>13 | 100<br>100 | 0.2<br>0.2 | Phenyl silanetriol 0.01<br>0.05 | 0.40<br>0.41 | 70<br>72 |
| 14<br>15 | 100<br>100 | 0.2<br>0.2 | Dodecyl silanetriol 0.01<br>0.05 | 0.45<br>0.44 | 68<br>72 |
| 16<br>17 | 100<br>100 | 0.2<br>0.2 | Cyclohexyl silanetriol 0.01<br>0.05 | 0.42<br>0.45 | 68<br>73 |
| 18<br>19 | 100<br>100 | 0.2<br>0.2 | Propenyl silanetriol 0.01<br>0.1 | 0.45<br>0.39 | 71<br>72 |
| 20<br>21 | 100<br>100 | 0.2<br>0.2 | Dimer of methyl silnetriol*) 0.01<br>0.05 | 0.35<br>0.40 | 80<br>75 |

*) 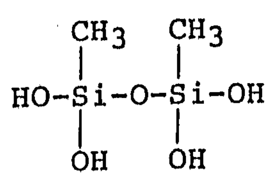

Example 3

Various compositions to which the crosslinking inhibitor for collagen in the present invention was added are shown below.

Example of Cream Composition I

| | Ingredient | Content wt part |
|---|---|---|
| Oil phase | Liquid paraffin | 6 |
| | Paraffin wax | 1 |
| | Isopropyl palmitate | 3 |
| | Spermaceti wax | 2 |
| | Cetyl alcohol | 2 |
| | Stearic acid | 2 |
| | Glycerin monostearate | 1.45 |
| | Polyoxyethylene (40) monostearate | 1.35 |
| | n-butyl-p-hydroxybenzoate | 0.1 |
| Water phase | Methyl p-hydroxybenzoate | 0.2 |
| | Aqueous soluton of 1% methyl silanetriol | 50 |
| | Purified water | 30.9 |

Example of Cream Composition II

| | Ingredient | Content wt part |
|---|---|---|
| Oil phase | Liquid paraffin | 6 |
| | Squalane | 3 |
| | Cetyl palmitate | 2 |
| | Cetyl alcohol | 2 |
| | Stearic acid | 2 |
| | n-butyl-p-hydroxybenzoate | 0.1 |
| Water phase | Methyl p-hydroxybenzoate | 0.1 |
| | Aqueous solution of 10% dimethyl silamediol | 50 |
| | Purified water | 34.8 |

Example of Cosmetic Lotion

| Ingredient | Content wt part |
|---|---|
| Aqueous solution of 0.05% of phenyl silanetriol | 40 |
| Propylene glycol | 5 |
| Ethanol | 5 |
| Disodium hydrogenphosphate | 0.05 |
| Methyl p-hydroxybenzoate | 0.1 |
| Perfume | 0.2 |
| Purified water | 49.65 |

Example of Ointment Composition

| | Ingredient | Content wt part |
|---|---|---|
| Oil phase | White vaseline | 25 |
| | Stearyl alcohol | 22 |
| | n-butyl-p-hydroxybenzoate | 0.015 |
| Water phase | Propylene glycol | 12 |
| | Sodium dodecyl sulfate | 1.5 |
| | Methyl p-hydroxybenzoate | 0.025 |
| | Aqueous solution of 2% methyl silanetriol | 25 |
| | Purified water | 14.46 |

Example of Ointment Composition

| | Ingredient | Content wt part |
|---|---|---|
| Oil phase | White vaseline | 40 |
| | Cetyl alcohol | 18 |
| | Sorbitane sesquiolate | 5 |
| | Polyoxyethylene (9) dodecylalcohol | 0.15 |
| | n-butyl-p-hydroxybenzoate | 0.1 |
| Water phase | Methyl-p-hydroxybenzoate | 0.1 |
| | Aqueous solution of 0.05% cyclohexyl silanetriol | 20 |
| | Purified water | 16.3 |

Example of Composition of Injection Solution

| Ingredient | Content wt part |
|---|---|
| Aqueous solution of 1% methyl silanetriol | 50 |
| Salt | 0.9 |
| Purified water | 49.1 |

Example of Composition of Health Drink

| Ingredient | Content wt part |
|---|---|
| Aqueous solution of 1% methyl silanetriol | 1 |
| Sugar | 10 |
| Perfume | Suitable amount |
| Purified water | 89 |

What is claimed is:

1. A crosslinking reaction inhibitor for collagen, which comprises a silicon compound having at least one silanol group in its molecule.

2. A crosslinking inhibitor according to Claim 1, wherein said silicon compound is a compound having the formula (I):

$$R_nSi(OH)_{4-n} \quad (I)$$

wherein R is an alkyl, aralkyl, alkenyl, cycloalkyl, or alkoxy group, two or more of R being the same or different from each other, and n is an integer of from 0 to 3 or a dehydrating condensation product thereof.

3. A crosslinking inhibitor according to Claim 1, wherein said silicon compound is methyl silanetriol.

4. A solution for injection containing a crosslinking reaction inhibitor for collagen as set forth in Claim 1.

5. Food containing a crosslinking reaction inhibitor for collagen as set forth in Claim 1.

6. A skin preparation containing a crosslinking reaction inhibitor for collagen as set forth in Claim 1.

FIG.1

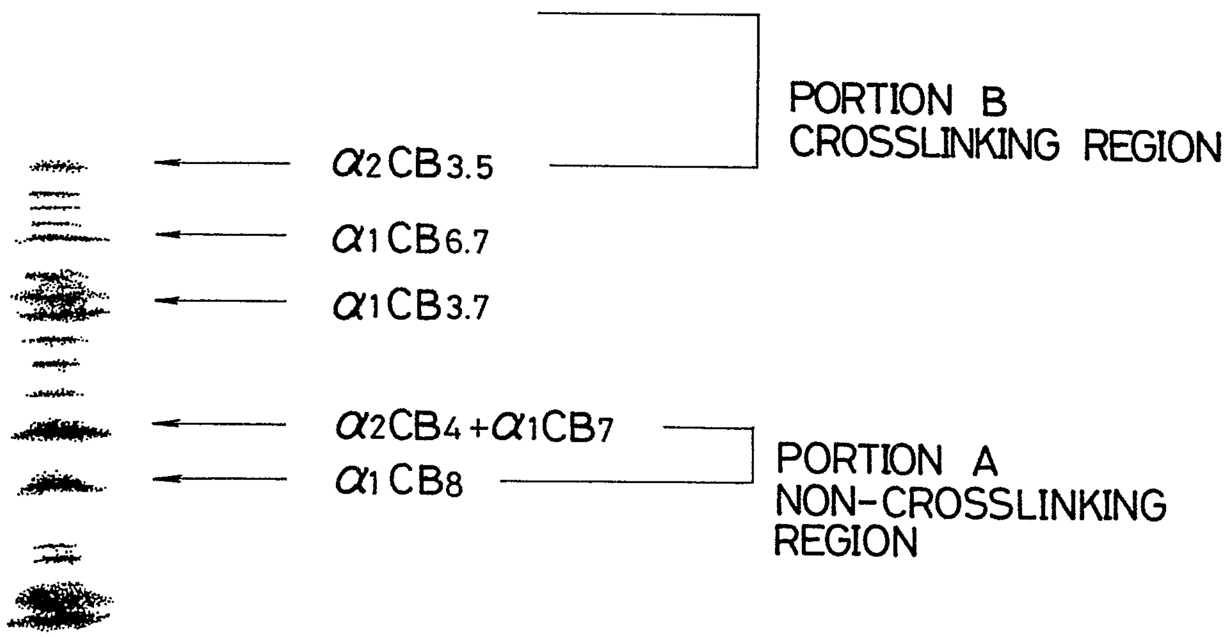
α2CB3.5
α1CB6.7
α1CB3.7
α2CB4+α1CB7
α1CB8
PORTION B
CROSSLINKING REGION
PORTION A
NON-CROSSLINKING
REGION

FIG.2